# EUROPÄISCHE PATENTSCHRIFT

(11) **EP 4 186 420 B1**
(45) Veröffentlichungstag und Bekanntmachung des Hinweises auf die Patenterteilung: **28.01.2026**
(21) Anmeldenummer: 21210213.1
(22) Anmeldetag: 24.11.2021
(51) Int. Cl.: A61B 5/11

(54) **KONTAKTSTREIFEN FÜR MATRATZE ODER MATRATZENUNTERLAGE**
CONTACT STRIP FOR MATTRESS OR MATTRESS UNDERLAY
BANDE DE CONTACT POUR MATELAS OU SOMMIERS

(43) Veröffentlichungstag der Anmeldung: 31.05.2023
(73) Patentinhaber: Acticom AG, 4147 Aesch (CH)
(72) Erfinder: Polat, Candemir, 4147 Eesch (CH)
(74) Vertreter: Latscha Schöllhorn Partner AG

(56) Entgegenhaltungen:
- EP-B1- 2 630 941
- US-A- 5 780 798
- US-A- 5 796 059

## Beschreibung

### Technisches Gebiet

Die Erfindung betrifft einen Kontaktstreifen für ein Auflageelement, wie etwa eine Matratze oder eine Matratzenunterlage, mittels welchem die Belegung einer Matratze durch einen Patienten detektierbar ist. Die Erfindung betrifft weiterhin ein entsprechendes Auflageelement mit zumindest einem Kontaktstreifen, sowie ein System bestehend aus einem Auflageelement und einer Kommunikationsvorrichtung.

Mittels derartiger Kontaktvorrichtungen wird regelmässig eine Überwachung der Belegung eines Bettes durch beispielsweise eine bettlägerige, pflegebedürftige, demente, desorientierte oder ggf. auch durch eine in intensivmedizinischer Behandlung befindliche Personen gewährleistet.

### Stand der Technik

Aus der CH 711 400 A2 ist eine Vorrichtung bekannt zur Überwachung einer in einem Bett liegenden oder einer an einem längsseitigen Bettrand sitzenden Person mit einem Eigengewicht. Die bekannte Vorrichtung umfasst einen Druckgeber, einen Druckschalter und einen Alarmgeber. Der Druckgeber wird hier aus Luftkammern, welche ein abgegrenztes Gesamtluftvolumen ausbilden, und der Person selbst gebildet und ist mit dem Alarmgeber verbunden.

Mittels einer solchen Vorrichtung kann zwar die Belegung (oder NichtBelegung) des entsprechenden Bettes unabhängig von der Liege- und/oder Sitzposition der Position im Bett auf zuverlässige Weise festgestellt werden, eine Bewegungsdetektion ist hiermit jedoch noch nicht möglich. Informationen hinsichtlich der Bewegung bzw. Nicht-Bewegung von Personen im Bett wären allerdings etwa für das Krankenhaus- oder Pflegepersonal von grossem Nutzen, da sie hierdurch erkennen könnten, wann die entsprechende Person beispielsweise gewendet oder umpositioniert werden muss. Zudem lösen die bekannten Vorrichtungen auch ein Alarmsignal aus, wenn die betreffende Person lediglich auf die Toilette geht, so dass auch in dieser Hinsicht ein Bedarf an Verbesserungen vorhanden ist.

Eine weitere Vorrichtung ist aus US5780798A bekannt.

Der vorliegenden Erfindung liegt daher die Aufgabe zu Grunde, eine Vorrichtung bereitzustellen, mittels welcher auf besonders sensitive Art und Weise auch Bewegungen einer Person im Bett detektiert werden können, sowie ein System mit einer solchen Vorrichtung welches über die Möglichkeit einer individuellen Einstellbarkeit verfügt.

### Darstellung der Erfindung

Die Aufgabe wird erfindungsgemäss durch einen Kontaktstreifen für ein Auflageelement, wie etwa eine Matratze oder eine Matratzenunterlage, gelöst, wie er im unabhängigen Anspruch 1 definiert ist. Vorteilhafte Ausführungsvarianten der Erfindung ergeben sich aus den abhängigen Ansprüchen.

Das Wesen der Erfindung besteht im Folgenden: einem Kontaktstreifen, welcher ein oberes Plattenelement und ein unteres Plattenelement aufweist, wobei zwischen dem oberen Plattenelement und dem unteren Plattenelement zumindest ein Dämpfungselement und zumindest ein Kontaktelement angeordnet sind.

Unter dem Begriff "Kontaktelement" wird vorliegend eine Vorrichtung verstanden, mittels welcher das Wirken bzw. Nicht-Wirken einer Gewichtskraft einer Person festgestellt werden kann. Dieses können grundsätzlich alle Formen von Schaltern oder Sensoren (wie beispielsweise piezoelektrische Sensoren oder aber Dehn-Mess-Streifen) sein.

Der Begriff "Dämpfungselement" umfasst vorliegend Vorrichtungen und/oder Materialien, welche insbesondere eine federartige Wirkung aufweisen, d.h. dass sie beim Auftreten einer Gewichtskraft diese etwas abbremsen bzw. das Wirken der vollen Gewichtskraft etwas verzögern können. Diesbezüglich soll die Vorrichtung bzw. das Material zum einen stauch- bzw. komprimierbar und andererseits wieder elastisch expandierbar sein (d.h. wenn die Gewichtskraft nicht mehr wirkt). In Betracht kommen alle Sorten von Federn bzw. Federelementen sowie alle geeigneten elastischen Kunststoffmaterialien.

Zwischen dem oberen Plattenelement und dem unteren Plattenelement ist ein mittleres Plattenelement angeordnet, welches zumindest eine Bohrung aufweist in welcher das zumindest eine Kontaktelement angeordnet ist.

Vorzugsweise ist das zumindest eine Dämpfungselement zwischen dem oberen Plattenelement und dem mittleren Plattenelement angeordnet. Mit anderen Worten ist das Dämpfungselement so zwischen dem oberem Plattenelement und Schalter positioniert, dass das zumindest eine Kontaktelement ausgelöst bzw. betätigt wird, wenn eine Person mit ihrem Gewicht bzw. einem Teil ihres Gewichts auf dem Kontaktstreifen liegt oder sitzt. Insbesondere im Fall von mehreren Kontaktelementen kann durch die Dämpfungswirkung des zumindest einen Dämpfungselements eine besonders vorteilhafte Sensibilität über den gesamten Kontaktstreifen bereitgestellt werden.

Vorzugsweise sind zwischen dem oberen Plattenelement und dem mittleren Plattenelement zwei Dämpfungselemente in Form von Schaumstoffstreifen angeordnet. Die Schaumstoffstreifen sind dabei vorzugsweise am linken rechten Rand des Kontaktstreifens angeordnet, d.h. parallel versetzt zu den Bohrungen. Auf diese Weise kann eine maximale Sensibilität erreicht werden. Als Dämpfungselemente können aber auch Federn oder anderweitige flexible Elemente zum Einsatz kommen.

Vorzugsweise weist das mittlere Plattenelement zwei bis sieben Bohrungen, vorzugsweise drei bis sechs Bohrungen und weiter vorzugsweise vier bis fünf Bohrungen auf, in welchen jeweils Kontaktelemente angeordnet sind. Die jeweils günstigste Anzahl an Bohrungen und Kontaktelementen hängt dabei etwa von der Grösse und Anzahl der verwendeten Kontaktstreifen ab sowie von der Grösse des Bettes.

Das zumindest eine Kontaktelement ist in Form eines Tast- oder Impulsschalters ausgebildet. Hierbei ist von Bedeutung, dass es sich um nicht-rastende Schalter handelt, welche nur eine geringe Grösse aufweisen (Miniaturschalter). Im Gegensatz zu etwa piezoelektrischen Sensoren benötigen solche Schalter nicht ständig Strom und sind ausserdem genauer.

Vorzugsweise sind die Kontaktelemente über Litzenelemente miteinander verbunden. Bei den Litzenelementen handelt es sich vorzugsweise um zwei parallele Kupferlitzen über welche die Kontaktelemente vorzugsweise in Serie geschaltet sind. An die Kupferlitzen schliessen sich bevorzugt elektrische Verbindungskabel an.

Vorzugsweise weisen das obere Plattenelement, das mittlere Plattenelement und/oder das untere Plattenelement eine Dicke von etwa 0.5 mm bis etwa 5 mm, vorzugsweise von etwa 1.0 mm bis etwa 3.0 mm und weiter vorzugsweise von etwa 1.5 mm bis etwa 2.5 mm auf. Auf diese Weise kann, in Abhängigkeit vom jeweils verwendeten Material, eine besonders vorteilhafte Flexibilität der einzelnen Plattenelemente des Kontaktstreifens eingestellt werden.

Vorzugsweise sind das obere Plattenelement, das mittlere Plattenelement und/oder das untere Plattenelement aus einem flexiblen bzw. biegsamen Material, bevorzugt aus Polypropylen, gebildet. Polypropylen weist gute Flexibilitätscharakteristika und ist zudem robust. Es sind allerdings auch andere Kunststoffmaterialen mit ähnlichen Eigenschaften denkbar. Grundsätzlich ist auch Holz als Material für die Plattenelemente vorstellbar. Die Plattenelemente sind zudem vorzugsweise formstabil und isolierend.

Ein weiterer Aspekt der vorliegenden Erfindung betrifft ein Auflageelement, welches vorzugsweise in Form einer Matratze oder einer Matratzenunterlage ausgebildet ist und welches zumindest einen erfindungsgemässen Kontaktstreifen aufweist.

Vorzugsweise weist das Auflageelement einen oberen Bereich, einen mittleren Bereich und einen unteren Bereich auf, in welchen jeweils ein bis drei Kontaktstreifen angeordnet sind. Auf diese Weise können die Belegung eines Bettes sowie Bewegungen des Patienten bzw. der Pflegeperson im Kopf-, Rumpf- und Beinbereich auf besonders zuverlässige Weise festgestellt werden.

Vorzugsweise weist das Auflageelement eine Verarbeitungseinheit auf, welche konfiguriert ist, eine Anzahl von Belastungswechseln pro Stunde bei den Kontaktstreifen zu registrieren und welche vorzugsweise konfiguriert ist, bei einer Unterschreitung einer Untergrenze für die Anzahl von Belastungswechseln pro Stunde ein (Alarm-)Signal zu generieren und/oder bei einer Überschreitung einer Obergrenze von Belastungswechseln pro Stunde ein (Alarm-)Signal zu generieren. Ein Belastungswechsel entspricht einer Bewegung einer Person auf dem Auflageelement. Ein Belastungswechsel wird insoweit festgestellt bzw. registriert, wenn zumindest ein Kontaktstreifen vom belasteten in den unbelasteten Zustand wechselt (Schalter wieder in Ruheposition) oder aber wenn zumindest ein Kontaktstreifen vom unbelasteten in den belasteten Zustand wechselt (Schalter niedergedrückt). Für die Registrierung bzw. Zählung und Auswertung der Belastungswechsel verfügt die Verarbeitungseinheit über eine entsprechende Software. Auf diese Weise wird eine besonders effiziente Überwachung der Bewegungen eines Patienten ermöglicht.

Vorzugsweise liegt die Untergrenze zwischen 2 und 20, vorzugsweise zwischen 5 und 15, noch bevorzugter zwischen 8 und 12 Belastungswechseln pro Stunde und wobei weiter vorzugsweise die Obergrenze zwischen 21 und 40, vorzugsweise zwischen 25 und 35, noch bevorzugter zwischen 28 und 32 Belastungswechseln pro Stunde liegt. Diese Werte haben sich in der Krankenhaus- und Pflegepraxis besonders bewährt.

Vorzugsweise weist das Auflageelement eine Verarbeitungseinheit auf, welcher konfiguriert ist mit einer Kommunikationsvorrichtung Daten auszutauschen. Diesbezüglich verfügt die Verarbeitungseinheit über einen entsprechenden Sender. Bei der Kommunikationsvorrichtung kann es sich beispielsweise um ein Mobiltelefon, ein Tablet, einen PC oder Ähnliches handeln. Bevorzugt werden allerdings tragbare Kommunikationsvorrichtungen, so dass das Krankenhaus- bzw. Pflegepersonal ortsunabhängig über die aktuellsten Informationen verfügt.

Ein weiterer Aspekt der vorliegenden Erfindung umfasst ein System aus einem erfindungsgemässen Auflageelement und einer Kommunikationsvorrichtung. Vorzugsweise können mittels der Kommunikationsvorrichtung die Untergrenze und die Obergrenze für die Belastungswechsel pro Stunde eingestellt werden sowie vorzugsweise auch eine Zeitverzögerung bzw. Verzögerungsintervalle für eine Meldung über eine Nichtbelegung des Auflageelements (kein Kontaktstreifen ist belastet). Weiter vorzugsweise beträgt diese Zeitverzögerung zwischen 1 Sekunde und 15 Minuten. Auf diese Weise kann etwa eine Toilettenpause für die jeweilige Person bzw. den jeweiligen Patienten eingestellt werden, ohne dass eine Meldung über eine Nichtbelegung des Bettes ausgelöst wird. Zudem können über die Kommunikationsvorrichtung Überwachungs- bzw. Beobachtungsintervalle (in welchen die Belastungswechsel bei den Kontaktstreifen (pro Stunde) registriert werden sollen) variabel eingestellt werden und zwar etwa in Zeiträumen von 1 Stunde, 2 Stunden, 3 Stunden oder mehr.

### Kurze Beschreibung der Zeichnungen

Weitere vorteilhafte Ausgestaltungen der Erfindung ergeben sich aus der nachfolgenden Beschreibung von Ausführungsbeispielen der Erfindung mit Hilfe der schematischen Zeichnung. Insbesondere wird im Folgenden der erfindungsgemässe Stuhl unter Bezugnahme auf die beigefügten Zeichnungen anhand von Ausführungsbeispielen detaillierter beschrieben. Es zeigen:
- Fig. 1:: eine Explosionsansicht eines Kontaktstreifens gemäss der vorliegenden Erfindung;
- Fig. 2:: eine perspektivische Ansicht eines teilweise zusammengebauten Kontaktstreifens gemäss der vorliegenden Erfindung; und
- Fig. 3:: eine schematische Ansicht eines Systems aus einem erfindungsgemässen Auflageelement zusammen mit einer Kommunikationsvorrichtung.

### Weg(e) zur Ausführung der Erfindung

Bestimmte Ausdrücke werden in der folgenden Beschreibung aus praktischen Gründen verwendet und sind nicht einschränkend zu verstehen. Die Wörter "rechts", "links", "unten" und "oben" bezeichnen Richtungen in der Zeichnung, auf die Bezug genommen wird. Die Ausdrücke "nach innen", "nach aussen" "unterhalb", "oberhalb", "links", "rechts" oder ähnliche werden zur Beschreibung der Anordnung bezeichneter Teile zueinander, der Bewegung bezeichneter Teile zueinander und der Richtungen hin zum oder weg vom geometrischen Mittelpunkt der Erfindung sowie benannter Teile derselben wie in den Figuren dargestellt verwendet. Diese räumlichen Relativangaben umfassen auch andere Positionen und Ausrichtungen als die in den Figuren dargestellten. Zum Beispiel wenn ein in den Figuren dargestelltes Teil umgedreht wird, sind Elemente oder Merkmale, die als "unterhalb" beschrieben sind, dann "oberhalb". Die Terminologie umfasst die oben ausdrücklich erwähnten Wörter, Ableitungen von denselben und Wörter ähnlicher Bedeutung.

Um Wiederholungen in den Figuren und der zugehörigen Beschreibung der verschiedenen Aspekte und Ausführungsbeispiele zu vermeiden, sollen bestimmte Merkmale als gemeinsam für verschieden Aspekte und Ausführungsbeispiele verstanden werden. Das Weglassen eines Aspekts in der Beschreibung oder einer Figur lässt nicht darauf schliessen, dass dieser Aspekt in dem zugehörigen Ausführungsbeispiel fehlt. Vielmehr kann ein solches Weglassen der Klarheit und dem Verhindern von Wiederholungen dienen. In diesem Zusammenhang gilt für die gesamte weitere Beschreibung folgende Festlegung: Sind in einer Figur zum Zweck zeichnerischer Eindeutigkeit Bezugszeichen enthalten, aber im unmittelbar zugehörigen Beschreibungstext nicht erwähnt, so wird auf deren Erläuterung in vorangehenden Figurenbeschreibungen Bezug genommen. Sind ausserdem im unmittelbar zu einer Figur gehörigen Beschreibungstext Bezugszeichen erwähnt, die in der zugehörigen Figur nicht enthalten sind, so wird auf die vorangehenden und nachstehenden Figuren verwiesen. Ähnliche Bezugszeichen in zwei oder mehreren Figuren stehen für ähnliche oder gleiche Elemente.

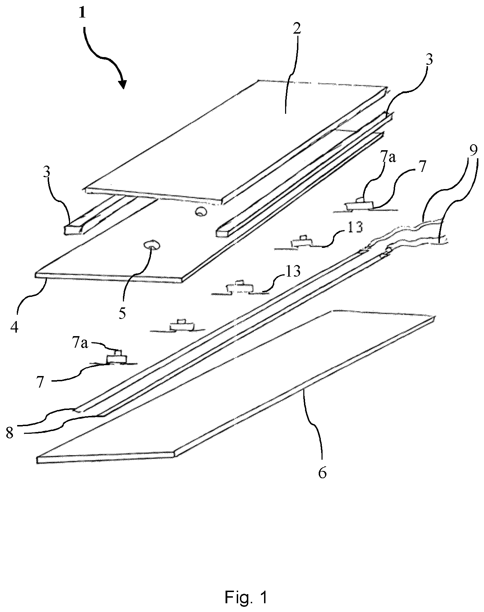

In **Fig. 1** wird eine Explosionsdarstellung einer beispielhaften Ausführungsform eines erfindungsgemässen Kontaktstreifens 1 veranschaulicht.

Zwischen dem unteren Plattenelement 6 und dem mittleren Plattenelement 4 mit den Bohrungen 5 sind zwei Kupferlitzen 8 angeordnet, über welche die hier gezeigten fünf Kontaktelemente bzw. Schalter 7 in Serie geschaltet werden. Dabei werden die Schalter 7 über Anschlusselemente 13 mit den Kupferlitzen 8 verbunden. An das hintere Ende der Kupferlitzen 8 schliessen sich elektrische Verbindungskabel 9 an, welche den Kontaktstreifen 1 ggf. mit anderen Kontaktstreifen und/oder mit einer Verarbeitungseinheit 11 verbinden. Die Bohrungen 5 in dem mittleren Plattenelement 4 sind so ausgestaltet, dass der Schalter 7 mit seinem Drücker 7a im montierten Zustand über die Oberfläche des mittleren Plattenelements 4 hervorragt.

Die beiden seitlichen Dämpfungselemente bzw. Schaumstoffstreifen 3 sorgen für eine ausreichende Beabstandung von dem oberen Plattenelement 2 zu dem mittleren Plattenelement 4, so dass, im unbelasteten Zustand, zwischen den Drückern 7a der Schalter 7 und der Unterseite des oberen Plattenelements 2 vorzugsweise ein kleiner Freiraum verbleibt. Die Bohrungen 5 in dem mittleren Plattenelement 4 sind vorzugsweise regelmässig beabstandet.

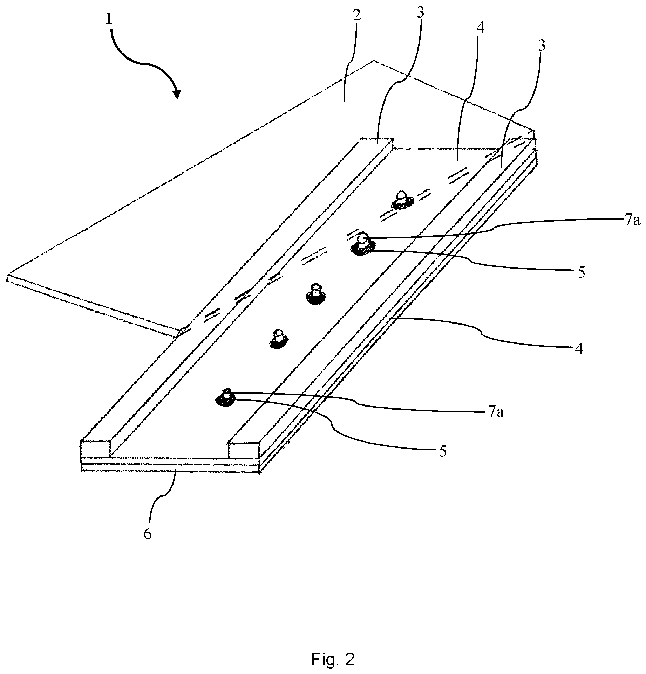

Die Anordnung der Schalter 7 in den Bohrungen 5 ist in **Fig. 2** gut zu erkennen. Bei der Montage werden die Schalter 7 von hinten bzw. unten her in die Bohrungen 5 eingeführt und werden an der Unterseite des mittleren Plattenelements 4 befestigt. Man erkennt wie die Drücker 7a über die Oberfläche des mittleren Plattenelements 4 hervorstehen. Die Schaumstoffstreifen 3 sind regemässig etwas höher als die Drücker 7a, zumindest aber gleich hoch wie die Drücker. Vorzugsweise weisen das obere Plattenelement 2, das mittlere Plattenelement 4 und das unter Plattenelement 6 dieselbe Dicke auf.

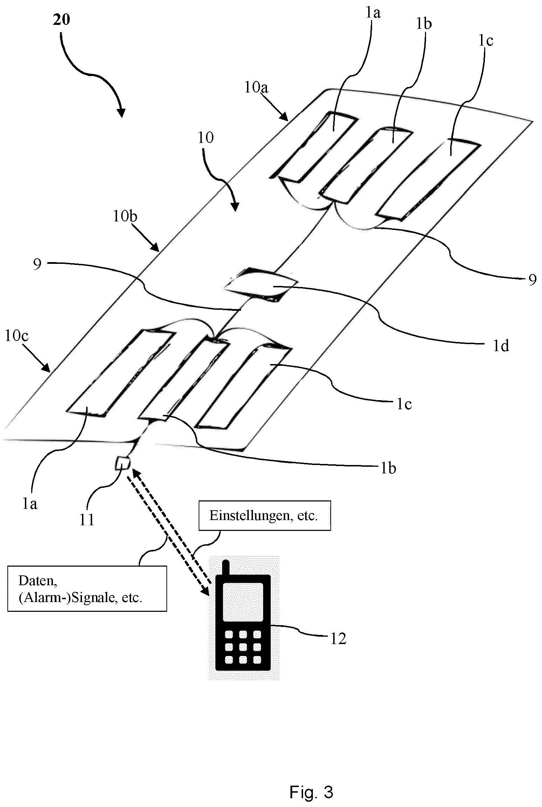

In **Fig. 3** wird schliesslich ein erfindungsgemässes Auflageelement 10 illustriert, welches etwa in Form einer Matratze oder einer Matratzenunterlage ausgebildet sein kann. Das Auflageelement 10 umfasst einen oberen Bereich 10a (z.B. Kopf- bzw. Schulterbereich), einen mittleren Bereich 10b (z.B. Gesässbereich) sowie einen unteren Bereich 10c (z.B. Fuss- bzw. Beinbereich). Im oberen Bereich 10a und im unteren Bereich 10c des Auflageelements 10 sind im vorliegenden Ausführungsbeispiel jeweils drei Kontaktstreifen 1a, 1b, 1c in longitudinaler Richtung parallel zueinander angeordnet. Im mittleren Bereich 10b des Auflageelements 10 ist ein Kontaktstreifen 1d angeordnet, welcher hier quer zu den Kontaktstreifen 1a, 1b, 1c im oberen Bereich und im unteren Bereich des Auflageelements 10 angeordnet ist. Die Anordnung der Kontaktstreifen 1a, 1b, 1c und 1d kann auch anderweitig ausgestaltet sein, allerdings ist die Anordnung vorzugsweise immer symmetrisch.

Die einzelnen Kontaktstreifen sind über Verbindungskabel 9 miteinander verbunden und parallel angeschlossen bzw. parallel geschaltet. Im vorliegenden Ausführungsbeispiel ist der mittlere Kontaktstreifen 1 des unteren Bereichs 10c des Auflageelements 10 mit einer Verarbeitungseinheit 11 verbunden. Die Verarbeitungseinheit 11 kann auch mit einem anderen Kontaktstreifen 1 verbunden sein; bevorzugt wird sie dort angeordnet wo normalerweise die (Not-)Rufvorrichtung des Patienten ist. Die Verarbeitungseinheit 11 ist derart konfiguriert, dass sie mit einer Kommunikationsvorrichtung 12 Daten austauschen kann und dass sie eine Be- sowie eine Entlastung der einzelnen Kontaktstreifen 1a, 1b, 1c, 1d registrieren kann. Die Verarbeitungseinheit 11 meldet an die Kommunikationsvorrichtung 12, wann ein Bett belegt ist bzw. wann ein Bett nicht mehr belegt ist sowie ggf. ausserdem wann und wie sich der Patient im Bett bewegt. Durch die Meldung der Bewegungen des Patienten bzw. der gepflegten Person im Bett (wann und wie) gewinnt das Pflege- oder Krankenhauspersonal beispielsweise Aufschluss darüber, wann der Patient bzw. die gepflegte Person gewendet oder umgelegt werden muss. Wenn die Schalter 7 der Kontaktstreifen 1a, 1b, 1c, 1d entlastet sind (also keine Gewichtskraft mehr auf sie wirkt), wird ein entsprechendes (Alarm-)Signal an die Kommunikationsvorrichtung 12 gesendet. Über die Kommunikationseinrichtung 12 kann aber auch manuell ein variables Verzögerungsintervall eingestellt werden, während dessen bei entlasteten Schaltern 7 der Kontaktstreifen 1a, 1b, 1c, 1d (d.h. bei Nichtbelegung des Betts) kein (Alarm-)Signal ausgelöst wird, um etwa Toilettenpausen Rechnung zu tragen. Die einzelnen Kontaktstreifen 1a, 1b, 1c, 1d können separat detektiert bzw. überwacht werden, so dass das Krankenhauspersonal oder die Pfleger präzise Informationen über Lageveränderungen des Patienten bzw. der gepflegten Person erhalten.

Anhand des in **Fig. 3** konkret gezeigten Auflageelements 10 soll im Folgenden eine bevorzugte Funktionsweise der erfindungsgemässen Überwachung erläutert werden. Eine Person im Ruhezustand belastet beispielsweise die Kontaktstreifen 1a, 1b, und 1c im oberen Kopf- bzw. Schulterbereich 10a wenn sie sich nicht bewegt. In dem Moment, wo sich die Person aber etwa auf die Seite dreht, entlastet sie beispielsweise die Kontaktstreifen 1a und 1b und belastet nur noch den Schalter 1c. Dieser Wechsel in der Belastung wird von der Verarbeitungseinheit 11 bzw. deren Software registriert und kann an die Kommunikationseinrichtung 12 gesendet werden. Gleiches gilt entsprechend für die Kontaktstreifen 1a, 1b, und 1c im unteren Fuss- bzw. Beinbereich 10c.

Für die Anwendung im Krankenhaus- und Pflegebereich hat sich als ein bevorzugtes Regime herausgestellt, dass solange eine Person sich 10 mal pro Stunde bewegt kein Alarm ausgelöst werden muss, da eine Umlagerung bzw. ein Wenden in diesem Fall nicht notwendig ist (d.h. 10 Belastungswechsel bei den Kontaktstreifen 1a, 1b, 1c, 1d pro Stunde als bevorzugte Untergrenze). Fällt die Anzahl der Bewegungen bzw. Belastungswechsel auf 9 oder darunter, wird ein Alarm ausgelöst, da sich die Person nicht ausreichend bewegt. Überschreitet die Anzahl der Bewegungen 30 pro Stunde nicht, wird davon ausgegangen, dass sich die Person in einem stabilen Zustand befindet, so dass kein Alarm ausgelöst werden muss. Wird jedoch die Anzahl von 30 Bewegungen bzw. Belastungswechseln pro Stunde überschritten, wird ein Alarm ausgelöst, da sich Person in einem unruhigen Zustand befindet (d.h. 30 Belastungswechsel bei den Kontaktstreifen 1a, 1b, 1c, 1d pro Stunde als bevorzugte Obergrenze).

Die Ober- bzw. Untergrenze können regelmässig in einem Bereich von 0 bis 100 Belastungswechsel pro Stunde variabel eingestellt werden. Zudem können die Überwachungsintervalle (in welchen die Belastungswechsel bei den Kontaktstreifen 1a, 1b, 1c, 1d registriert werden sollen) variabel eingestellt werden in Zeiträume von 1 Stunde, 2 Stunden, 3 Stunden oder mehr.

Wenn der Kontaktstreifen 1d im mittleren Gesässbereich 10b von Be- auf Entlastung wechselt (d.h. insbesondere wenn der Kontaktstreifen 1d zuvor alleiniger belasteter Kontaktstreifen war), ist dies regelmässig ein Hinweis darauf, dass die Person aus dem Bett ausgestiegen ist, so dass ein Ausstiegsalarm, ggf. nach einer voreingestellten Verzögerung, ausgelöst werden kann.

Generell kann die Verarbeitungseinrichtung 11 jeden Kontaktstreifen 1a, 1b, 1c, 1d separat detektieren, entsprechende Belastungsdaten mittels einer Software verarbeiten und so entsprechende Bewegungsdaten sowie ggf. entsprechende (Alarm-) Signale an die Kommunikationsvorrichtung 12 senden. Über die Kommunikationsvorrichtung 12 können sämtliche benötigten Einstellungen vorgenommen werden, wie beispielsweise die Unter- bzw. Obergrenzen für die Bewegungen bzw. Belastungswechsel sowie entsprechende Beobachtungsintervalle von variabler Zeitdauer (d.h. ein, zwei, drei oder mehr Stunden) und darüber hinaus auch variable Verzögerungsintervalle für mögliche Toilettenpausen während derer etwa kein (Alarm-)Signal ausgelöst wird. Dies sind allerdings nur einige Beispiele; weiterer Datenaustausch zwischen der Verarbeitungseinrichtung 11 und der Kommunikationsvorrichtung 12 ist möglich.

Im Weiteren schliesst der Ausdruck "umfassen" und Ableitungen davon andere Elemente oder Schritte nicht aus. Ebenfalls schliesst der unbestimmte Artikel "ein" bzw. "eine" und Ableitungen davon eine Vielzahl nicht aus. Die Funktionen mehrerer in den Ansprüchen aufgeführter Merkmale können durch eine Einheit beziehungsweise einen Schritt erfüllt sein. Die blosse Tatsache, dass bestimmte Masse in zueinander verschiedenen abhängigen Ansprüchen aufgeführt sein können, bedeutet nicht, dass eine Kombination dieser Masse nicht vorteilhaft verwendet werden kann. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Die Begriffe "etwa" und "ungefähr" im Zusammenhang mit einem gegebenen Zahlenwert oder -bereich kann sich auf einen Wert beziehungsweise Bereich beziehen, der innerhalb 20%, innerhalb 10%, innerhalb 5% oder innerhalb 2% des gegebenen Werts beziehungsweise Bereichs liegt.

### Liste der Bezugszeichen:

- 1a-d: Kontaktstreifen
- 2: oberes Plattenelement
- 3: Dämpfungselement
- 4: mittleres Plattenelement
- 5: Bohrungen (mittlere Platte)
- 6: unteres Plattenelement
- 7: Kontaktelemente/Schalter
- 7a: Drücker
- 8: Litzenelemente
- 9: elektrische Leitungen/Verbindungskabel
- 10: Auflageelement (z.B. Matratze oder Matratzenunterlage)
- 10a: oberer Bereich
- 10b: mittlerer Bereich
- 10c: unterer Bereich
- 11: Verarbeitungseinheit
- 12: Kommunikationsvorrichtung
- 13: Anschlusselemente
- 20: System

## Patentansprüche

1. Kontaktstreifen (1, 1a, 1b, 1c, 1d) für ein Auflageelement (10), wie etwa eine Matratze oder eine Matratzenunterlage, aufweisend ein oberes Plattenelement (2) und ein unteres Plattenelement (6), wobei zwischen dem oberen Plattenelement (2) und dem unteren Plattenelement (6) zumindest ein Dämpfungselement (3) und zumindest ein Kontaktelement (7) angeordnet sind, wobei zwischen dem oberen Plattenelement (2) und dem unteren Plattenelement (6) ein mittleres Plattenelement (4) angeordnet ist, welches zumindest eine Bohrung (5) aufweist in welcher das zumindest eine Kontaktelement (7) angeordnet ist, das zumindest eine Kontaktelement (7) in Form eines Tast- oder Impulsschalters ausgebildet ist, **dadurch gekennzeichnet, dass** die Bohrungen (5) in dem mittleren Plattenelement (4) so ausgestaltet sind, dass der Tast- oder Impulsschalter (7) mit seinem Drücker (7a) im montierten Zustand über die Oberfläche des mittleren Plattenelements (4) hervorragt.

2. Kontaktstreifen (1, 1a, 1b, 1c, 1d) gemäss Anspruch 1, wobei das zumindest eine Dämpfungselement (3) zwischen dem oberen Plattenelement (2) und dem mittleren Plattenelement (4) angeordnet ist.

3. Kontaktstreifen (1, 1a, 1b, 1c, 1d) gemäss einem der Ansprüche 1 bis 2, wobei zwischen dem oberen Plattenelement (2) und dem mittleren Plattenelement (4) zwei Dämpfungselemente (3) in Form von Schaumstoffstreifen angeordnet sind.

4. Kontaktstreifen (1, 1a, 1b, 1c, 1d) gemäss einem der Ansprüche 1 bis 3, wobei das mittlere Plattenelement (4) zwei bis sieben Bohrungen (5), vorzugsweise drei bis sechs Bohrungen (5) und weiter vorzugsweise vier bis fünf Bohrungen (5) aufweist, in welchen jeweils Kontaktelemente (7) angeordnet sind.

5. Kontaktstreifen (1, 1a, 1b, 1c, 1d) gemäss einem der vorhergehenden Ansprüche, wobei die Kontaktelemente (7) über Litzenelemente (8) miteinander verbunden sind.

6. Kontaktstreifen (1, 1a, 1b, 1c, 1d) gemäss einem der vorhergehenden Ansprüche, wobei das obere Plattenelement (2), das mittlere Plattenelement (4) und/oder das untere Plattenelement (6) eine Dicke von etwa 0.5 mm bis etwa 5 mm, vorzugsweise von etwa 1.0 mm bis etwa 3.0 mm und weiter vorzugsweise von etwa 1.5 mm bis etwa 2.5 mm aufweisen.

7. Kontaktstreifen (1, 1a, 1b, 1c, 1d) gemäss einem der vorhergehenden Ansprüche, wobei das obere Plattenelement (2), das mittlere Plattenelement (4) und/oder das untere Plattenelement (6) aus einem flexiblen Material, vorzugsweise aus Polypropylen, gebildet sind.

8. Auflageelement (10), insbesondere in Form einer Matratze oder einer Matratzenunterlage, aufweisend zumindest einen Kontaktstreifen (1, 1a, 1b, 1c, 1d) nach einem der Ansprüche 1 bis 7.

9. Auflageelement (10) gemäss Anspruch 8, wobei das Auflageelement (10) einen Kopf- bzw. Schulterbereich (10a), einen Gesässbereich und einen Fuss- bzw. Beinbereich (10c) aufweist, in welchen jeweils ein bis drei Kontaktstreifen (1, 1a, 1b, 1c, 1d) angeordnet sind.

10. Auflageelement (10) gemäss Anspruch 8 oder 9, wobei das Auflageelement eine Verarbeitungseinheit (11) aufweist, welche konfiguriert ist, eine Anzahl von Belastungswechseln pro Stunde bei den Kontaktstreifen (1, 1a, 1b, 1c, 1d) zu registrieren und welche vorzugsweise konfiguriert ist, bei einer Unterschreitung einer Untergrenze für die Anzahl von Belastungswechseln pro Stunde ein (Alarm-)Signal zu generieren und/oder bei einer Überschreitung einer Obergrenze von Belastungswechseln pro Stunde ein (Alarm-)Signal zu generieren.

11. Auflageelement (10) gemäss Anspruch 10, wobei die Untergrenze zwischen 2 und 20, vorzugsweise zwischen 5 und 15, noch bevorzugter zwischen 8 und 12 Belastungswechseln pro Stunde liegt und wobei weiter vorzugsweise die Obergrenze zwischen 21 und 40, vorzugsweise zwischen 25 und 35, noch bevorzugter zwischen 28 und 32 Belastungswechseln pro Stunde liegt.

12. Auflageelement (10) gemäss einem der Ansprüche 8 bis 11, wobei die Verarbeitungseinheit (11) konfiguriert, ist mit einer Kommunikationsvorrichtung (12) Daten auszutauschen.

13. System (20) aus einem Auflageelement (10) gemäss Anspruch 12 und einer Kommunikationsvorrichtung (12).

## Claims

1. Contact strip (1, 1a, 1b, 1c, 1d) for a support element (10), such as a mattress or a mattress underlay, comprising an upper plate element (2) and a lower plate element (6), wherein at least one damping element (3) and at least one contact element (7) are arranged between the upper plate element (2) and the lower plate element (6), wherein a middle plate element (4) is arranged between the upper plate element (2) and the lower plate element (6) which has at least one bore (5) in which the at least one contact element (7) is arranged, the at least one contact element (7) is designed in the form of a touch switch or pulse switch, **characterised in that** the bores (5) in the middle plate element (4) are designed such that the touch switch or pulse switch (7) protrudes with its push button (7a) above the surface of the middle plate element (4) when assembled.

2. Contact strip (1, 1a, 1b, 1c, 1d) according to claim 1, wherein the at least one damping element (3) is arranged between the upper plate element (2) and the middle plate element (4).

3. Contact strip (1, 1a, 1b, 1c, 1d) according to any one of claims 1 to 2, wherein two damping elements (3) in the form of foam strips are arranged between the upper plate element (2) and the middle plate element (4).

4. Contact strip (1, 1a, 1b, 1c, 1d) according to any one of claims 1 to 3, wherein the middle plate element (4) has two to seven bores (5), preferably three to six bores (5) and more preferably four to five bores (5), in each of which contact elements (7) are arranged.

5. Contact strip (1, 1a, 1b, 1c, 1d) according to any one of the preceding claims, wherein the contact elements (7) are connected to each other via stranded elements (8).

6. Contact strip (1, 1a, 1b, 1c, 1d) according to any one of the preceding claims, wherein the upper plate element (2), the middle plate element (4) and/or the lower plate element (6) have a thickness of approximately 0.5 mm to approximately 5 mm, preferably approximately 1.0 mm to approximately 3.0 mm and more preferably approximately 1.5 mm to approximately 2.5 mm.

7. Contact strip (1, 1a, 1b, 1c, 1d) according to any one of the preceding claims, wherein the upper plate element (2), the middle plate element (4) and/or the lower plate element (6) are formed from a flexible material, preferably polypropylene.

8. Support element (10), in particular in the form of a mattress or mattress underlay, comprising at least one contact strip (1, 1a, 1b, 1c, 1d) according to any one of claims 1 to 7.

9. Support element (10) according to claim 8, wherein the support element (10) has a head or shoulder area (10a), a buttocks area and a foot or leg area (10c), in each of which one to three contact strips (1, 1a, 1b, 1c, 1d) are arranged.

10. Support element (10) according to claim 8 or 9, wherein the support element has a processing unit (11) which is configured to register a number of load changes per hour at the contact strips (1, 1a, 1b, 1c, 1d) and which is preferably configured to generate an (alarm) signal when the number of load changes per hour falls below a lower limit and/or to generate an (alarm) signal when the number of load changes per hour exceeds an upper limit.

11. Support element (10) according to claim 10, wherein the lower limit is between 2 and 20, preferably between 5 and 15, more preferably between 8 and 12 load changes per hour, and wherein the upper limit is preferably between 21 and 40, preferably between 25 and 35, and even more preferably between 28 and 32 load changes per hour.

12. Support element (10) according to one of claims 8 to 11, wherein the processing unit (11) is configured to exchange data with a communication device (12).

13. System (20) comprising a support element (10) according to claim 12 and a communication device (12).

## Revendications

1. Bande de contact (1, 1a, 1b, 1c, 1d) pour un élément d'appui (10), tel qu'un matelas ou un support pour matelas, présentant un élément formant plaque supérieur (2) et un élément formant plaque inférieur (6), dans laquelle au moins un élément d'amortissement (3) et au moins un élément de contact (7) sont disposés entre l'élément formant plaque supérieur (2) et l'élément formant plaque inférieur (6), dans laquelle un élément formant plaque intermédiaire (4) est disposé entre l'élément formant plaque supérieur (2) et l'élément formant plaque inférieur (6), lequel élément formant plaque intermédiaire présente au moins un alésage (5) dans lequel est disposé l'au moins un élément de contact (7), l'au moins un élément de contact (7) étant réalisé sous forme de commutateur à action fugitive ou à impulsion, **caractérisé en ce que** les alésages (5) dans l'élément formant plaque intermédiaire (4) sont conçus de sorte que, à l'état monté, le commutateur à action fugitive ou à impulsion (7) fait saillie, avec son poussoir (7a), de la surface de l'élément formant plaque intermédiaire (4).

2. Bande de contact (1, 1a, 1b, 1c, 1d) selon la revendication 1, dans laquelle l'au moins un élément d'amortissement (3) est disposé entre l'élément formant plaque supérieur (2) et l'élément formant plaque intermédiaire (4).

3. Bande de contact (1, 1a, 1b, 1c, 1d) selon l'une des revendications 1 à 2, dans laquelle deux éléments d'amortissement (3) sous forme de bandes de mousse sont disposés entre l'élément formant plaque supérieur (2) et l'élément formant plaque intermédiaire (4).

4. Bande de contact (1, 1a, 1b, 1c, 1d) selon l'une des revendications 1 à 3, dans laquelle l'élément formant plaque intermédiaire (4) présente deux à sept alésages (5), de préférence trois à six alésages (5) et plus préférablement quatre à cinq alésages (5) dans lesquels sont disposés respectivement des éléments de contact (7).

5. Bande de contact (1, 1a, 1b, 1c, 1d) selon l'une des revendications précédentes, dans laquelle les éléments de contact (7) sont connectés entre eux par des éléments formant torons (8).

6. Bande de contact (1, 1a, 1b, 1c, 1d) selon l'une des revendications précédentes, dans laquelle l'élément formant plaque supérieur (2), l'élément formant plaque intermédiaire (4) et/ou l'élément formant plaque inférieur (6) présentent une épaisseur d'environ 0,5 mm à environ 5 mm, de préférence d'environ 1,0 mm à environ 3,0 mm, et plus préférablement d'environ 1,5 mm à environ 2,5 mm.

7. Bande de contact (1, 1a, 1b, 1c, 1d) selon l'une des revendications précédentes, dans laquelle l'élément formant plaque supérieur (2), l'élément formant plaque intermédiaire (4) et/ou l'élément formant plaque inférieur (6) sont formés en un matériau flexible, de préférence en polypropylène.

8. Élément d'appui (10), en particulier sous forme de matelas ou de support pour matelas, présentant au moins une bande de contact (1, 1a, 1b, 1c, 1d) selon l'une des revendications 1 à 7.

9. Élément d'appui (10) selon la revendication 8, dans lequel l'élément d'appui (10) présente une zone pour tête ou épaules (10a), une zone pour fesses et une zone pour pieds ou jambes (10c) dans lesquelles sont disposées respectivement une à trois bandes de contact (1, 1a, 1b, 1c, 1d).

10. Élément d'appui (10) selon la revendication 8 ou 9, dans lequel l'élément d'appui présente une unité de traitement (11) qui est configurée pour enregistrer un certain nombre de changements de charge par heure au niveau des bandes de contact (1, 1a, 1b, 1c, 1d) et qui est de préférence configurée pour générer un signal (d'alarme) lorsqu'une limite inférieure pour le nombre de changements de charge par heure n'est pas atteinte et/ou pour générer un signal (d'alarme) lorsqu'une limite supérieure de changements de charge par heure est dépassée.

11. Élément d'appui (10) selon la revendication 10, dans lequel la limite inférieure est comprise entre 2 et 20, de préférence entre 5 et 15, plus préférablement entre 8 et 12 changements de charge par heure et dans lequel la limite supérieure est encore plus préférablement comprise entre 21 et 40, de préférence entre 25 et 35, plus préférablement entre 28 et 32 changements de charge par heure.

12. Élément d'appui (10) selon l'une des revendications 8 à 11, dans lequel l'unité de traitement (11) est configurée pour échanger des données avec un dispositif de communication (12).

13. Système (20) constitué d'un élément d'appui (10) selon la revendication 12 et d'un dispositif de communication (12).
